# EUROPEAN PATENT SPECIFICATION

(11) **EP 3 193 859 B1**
(45) Date of publication and mention of the grant of the patent: **05.07.2023**
(21) Application number: 15841584.4
(22) Date of filing: 15.09.2015
(51) Int. Cl.: A61K 9/48, A61K 9/22, A61K 31/198, A61P 37/08

(54) **PREPARATIONS FOR TREATMENT AND PREVENTION OF ALCOHOL FLUSHING AND ALCOHOL-INDUCED HYPERSENSITIVITY REACTIONS**
PRÄPARATE ZUR BEHANDLUNG UND PRÄVENTION VON ALKOHOL-FLUSHING UND ALKOHOLINDUZIERTEN ÜBEREMPFINDLICHKEITSREAKTIONEN
PRÉPARATIONS POUR LE TRAITEMENT ET LA PRÉVENTION DE LA RÉACTION D'INTOLÉRANCE À L'ALCOOL ("ALCOHOL FLUSHING") ET DE RÉACTIONS D'HYPERSENSIBILITÉ INDUITES PAR L'ALCOOL

(30) Priority: 15.09.2014 FI 20145806
(43) Date of publication of application: 26.07.2017
(73) Proprietor: BIOHIT OYJ, 00880 Helsinki (FI)
(72) Inventor: KORPELA, Semi, FI-00880 Helsinki (FI); SYRJÄNEN, Kari, FI-00880 Helsinki (FI); SALASPURO, Mikko, FI-00290 Helsinki (FI); SUOVANIEMI, Osmo, FI-00570 Helsinki (FI)
(74) Representative: Laine IP Oy
(86) International application number: PCT/FI2015/050612
(87) International publication number: WO 2016/042205

(56) References cited:
- EP-A1- 2 589 381
- WO-A1-2013/178880
- WO-A1-2014/011676
- WO-A1-2014/143192
- WO-A2-2012/027603
- US-A1- 2014 256 760
- US-A1- 2015 202 181
- A. LINNEBERG ET AL: "Prevalence of self-reported hypersensitivity symptoms following intake of alcoholic drinks", CLINICAL & EXPERIMENTAL ALLERGY : JOURNAL OF THE BRITISH SOCIETY FOR ALLERGY AND CLINICAL IMMUNOLOGY, vol. 38, no. 0, 10 October 2007 (2007-10-10), pages 145-151, XP55458104, UK ISSN: 0954-7894, DOI: 10.1111/j.1365-2222.2007.02837.x

## Description

### Technical field

The present invention relates to the prevention and/or treatment of effects associated with alcohol consumption and more specifically to compositions for the prevention or treatment of alcohol-induced flushing and hypersensitivity reactions.

### Background

Alcohol flush reaction (also known as Asian flush syndrome, Asian flush, Asian glow, among others) is a condition in which an individual develops flushes or blotches associated with erythema on the face, neck, shoulders, and, in some cases, the entire body after consuming alcoholic beverages. The reaction is the result of an accumulation of acetaldehyde, a metabolic byproduct of the catabolic metabolism of alcohol, and is caused by an aldehyde dehydrogenase deficiency.

Based on strong scientific evidence this syndrome has been associated with an increased risk of oral, pharyngeal, esophageal and gastric cancer in those who drink alcohol. It has also been associated with lower than average rates of alcoholism, possibly due to its association with adverse effects after drinking alcohol.

Metabolism of alcohol takes place mainly in the liver and involves a two-step enzymatic reaction. First, alcohol is oxidized to acetaldehyde by cytoplasmic alcohol dehydrogenase (ADH) enzyme. Secondly, acetaldehyde is oxidized to acetic acid by mitochondrial and cytoplasmic aldehyde dehydrogenase (ALDH) enzymes. Acetic acid is then transported from the liver to the muscles and adipose tissue where it is further broken down into carbon dioxide and water. Thus, acetaldehyde concentrations in blood and tissues are regulated by balance of both alcohol - and ALDH enzymes. This is important, because although ethanol is toxic to the body, acetaldehyde is much more toxic than ethanol. Furthermore, acetaldehyde derived from alcoholic beverages has been classified as a Group 1 carcinogen for humans by the International Agency for Research on Cancer (IARC/WHO)(IARC 2012).

In the majority of humans, the capacity of liver to eliminate acetaldehyde formed from ethanol is so efficient that measurable levels of acetaldehyde do not escape from the liver to blood circulation. However, the situation may be different in other tissues. For instance, in the digestive tract both microbes and mucosal cells are able to produce locally acetaldehyde from ethanol, but arc not able to eliminate it. Therefore, in the presence of ethanol, significant amounts of acetaldehyde accumulate in the oral cavity, esophagus, stomach and large intestine (Salaspuro 2003, 2009, 2011). In fact, the highest levels of ethanol-derived acetaldehyde are found in the digestive tract.

Approximately 36% of East Asians (Japanese, Chinese, Taiwanese and Koreans) show a characteristic physiological response to drinking alcohol that include facial flushing, increased skin temperature, tachycardia, decrease of diastolic blood pressure and sometimes also nausea (Kupari *et al.* 1983, Väkeväinen *et al.* 2001, Brooks *et al.* 2009). This so-called alcohol flushing response (also known as "Asian flush" or "Asian glow") is predominantly due to an inherited deficiency of the main enzyme for acetaldehyde oxidation, mitochondrial aldehyde dehydrogenase 2 (ALDH2). It has been estimated that there are at least 540 million ALDH2-deficient individuals in the world representing approximately 8% of the global population.

In East Asians (Orientals), the mechanism of Asian flush and hypersensitivity reactions e.g. bronchoconstriction in human asthmatics, has been proposed to be elevated blood levels of acetaldehyde, secondary to decreased elimination by deficient ALDH2-enzyme. Acetaldehyde has been shown to enhance histamine release from human and rat mast cells (Shimoda *et al.* 1996, Koivisto *et al.* 1999). The same mechanism may be involved also in the pathogenesis of allergic rhinitis (hay fever), allergic conjunctivitis, atopic dermatitis, eosinophilic esophagitis, anaphylaxis, chronic bronchitis, and chronic obstructive pulmonary disease (COPD) (Nihlen *et al.* 2005, Linneberg *et al.* 2008). This concept is strongly supported by the observations that histamine receptor antagonists (H2 blockers) and drugs inhibiting histamine release do alleviate alcohol-related hypersensitivity reactions (Miller *et al.* 1988, Myou *et al.* 1995, Takao *et al.* 1999). Furthermore, combined use of H₁₋ and H₂ receptor antagonists has been shown to neutralize alcohol-induced cutaneous flushing and systolic hypotension in Orientals (Miller *et al.* 1998).

More recently, alcohol-related and genetically determined hypersensitivity reactions have been described also in Caucasian populations (Linneberg *et al.* 2008). Indeed, 14% of the general adult population in Denmark had experienced hypersensitivity symptoms from the nose, lungs, or skin following intake of alcoholic drinks. These reactions were proposed to be caused by a mechanism similar to that described in East Asians, i.c., histaminc-rclcasing effect of acetaldehyde (Linneberg *et al.* 2008). These alcohol hypersensitivity reactions were found to be associated with a genetically determined fast metabolism of ethanol i.e., enhanced production of acetaldehyde - the A allele of ADHlb and ALDH1b1, the function of which is not yet known (Linneberg *et al.* 2010).

In subjects with normal ALDH2-enzyme, alcohol drinking does not result in detectable levels of free acetaldehyde in peripheral blood (Lindroos *et al.* 1980). However, after a small or moderate dose of alcohol, markedly high acetaldehyde levels are found in the saliva (Homann *et al.* 1997, Väkeväinen *et al.* 2000). This is by and large due to oral microbes and mucosal cells that possess significant ADH activity metabolizing ethanol to acetaldehyde locally (Homann *et al.* 1997, Dong *et al.* 1996), but which are not able to eliminate it (Salaspuro 2003, 2009).

In contrast to Caucasians, slightly elevated blood acetaldehyde levels after alcohol intake have been demonstrated among ALDH2-deficient East-Asians, and this has been suggested to provide the basis for their flushing, hypersensitivity reactions and cardiovascular responses seen after alcohol intake (Mizoi *et al.* 1979, Chen *et al.* 2009). However, in ALDH2-deficient individuals, salivary acetaldehyde levels after a single dose of alcohol are 10 - 20 times higher than those in the blood, and 2-3 times higher than in individuals with normal ALDH2-enzyme (Väkeväinen *et al.* 2000, Yokoyama *et al.* 2008). Furthermore, after intragastric infusion of alcohol, ALDH2- deficient subjects have 5-6 times higher acetaldehyde levels in their gastric juice than those with normal ALDH2-enzyme.

The significant effects of local acetaldehyde in the G-I tract and consequently in the pathogenesis of flushing and hypersensitivity reactions is supported also by other evidence. Accordingly, in ALDH2-deficient individuals, a potent inhibitor of ADH enzyme (4-methylpyrazole, 4-MP) has been shown to markedly decrease alcohol-induced increase in salivary acetaldehyde levels (Väkeväinen *et al.* 2001). In these persons, 4-MP suppressed also significantly the flushing response to alcohol. Similarly, the increase in heart rate and skin temperature as well as the drop in diastolic blood pressure disappeared when such persons used 4-MP before ethanol intake (Väkeväinen *et al.* 2001).

Based on the above discussed observations, it is plausible that the acetaldehyde-mediated flushing and hypersensitivity reactions after alcohol intake are not caused by elevated blood acetaldehyde levels. In contrast, local acetaldehyde- mediated release of histamine from the tissue mast cells in the upper digestive tract mucosa could be the major mechanism explaining these reactions. It is a common knowledge that upper G-I-tract contains abundantly histamine releasing mast cells (Syrjänen 1975, Steer 1976, Walsh 2003).

WO 2012/027603 relates to therapeutic consumable compositions for reducing facial flush effect incident to alcohol consumption in some persons of Asian descent or persons with ALDH2 gene deficiency. The composition is a single dose and designed for lowering blood alcohol level and removing acetaldehyde from the liver.

WO 2014/011676 and US 2014/256760 are directed to compositions comprising for example N-acetylcysteine for reducing alcohol reaction and relating unpleasant symptoms that accompany the consumption of alcoholic beverages in a subject. Both applications are, however, silent about histamine-releasing effect of locally formed acetaldehyde.

### Summary of the Invention

The present invention relates to cysteine-releasing pharmaceutical preparations for use in preventing and/or treating deleterious effects associated with alcohol consumption (i.e. in harm reduction). The preparations include for example slowly L-cysteine releasing preparations either in the mouth or stomach.

More precisely, the present invention relates to a preparation for use as defined in claim 1.

One major advantage of the present invention is that alcohol-induced flushing and hypersensitivity reactions can be markedly prevented and treated by eliminating and inactivating acetaldehyde by using preparation that release cysteine in the stomach or in oral cavity, as herein disclosed.

Next, the invention is described by the following figures and embodiments.

### Short description of Figures

Figure 1: Chemical formula illustrating the covalent binding of acetaldehyde to L-cysteine. As a result, an inactive stable product, 2-methylthiazolidine-4-carboxylic acid (MTCA) is formed.

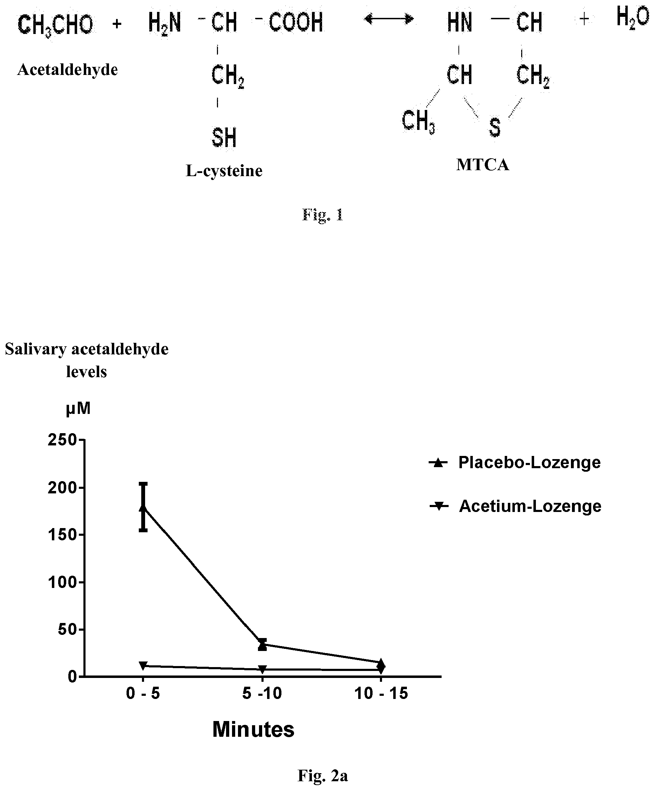

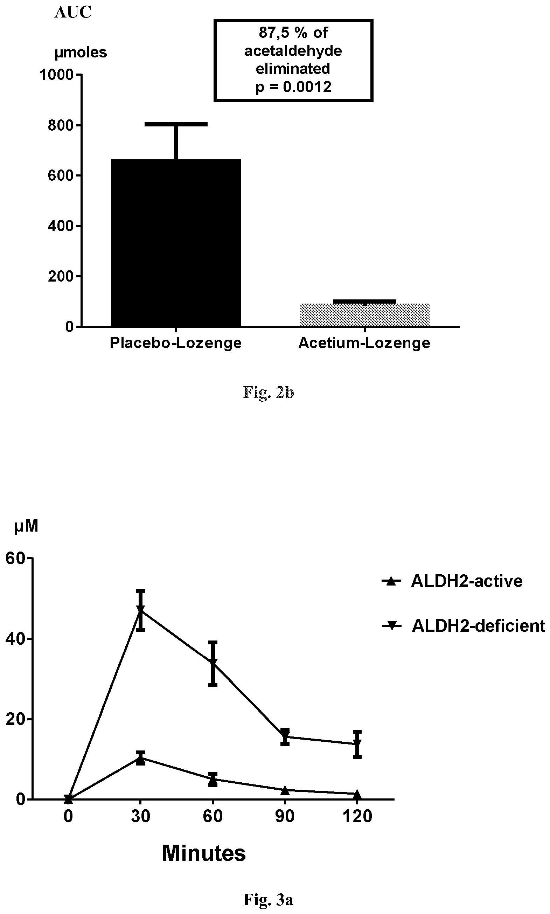

**Figures 2a** **and** **2b**: The effect of slowly L-cysteine releasing Acetium-Lozenge on acetaldehyde levels in saliva (a) and areas under the curve (b)(means±SEM). Four volunteers sucked one Acetium-Lozenge containing either 3 mg L-cysteine or placebo. Thereafter they rinsed their mouths with 5ml of Grappa (Grappa Di Monovitigino, acetaldehyde 5344 µM, alcohol 35.6 vol %) for 5 seconds. Grappa was spitted off and volunteers took another Acetium-Lozenge and sucked it for the rest of the experiment. Thereafter whole saliva was collected as pooled samples for following 0 - 5, 5 -10 and 10 - 15 minutes and their acetaldehyde and ethanol concentrations were determined by head space gas chromatography.
**Figures 3a** **and** **3b**: The effect of ALDH2-deficiency on gastric juice acetaldehyde levels (a) and areas under the curve (b) after intragastric infusion of ethanol (15 vol%, 0.5 g/kg) (means f SEM). 10 ALDH2-active and 10 ALDH2-negative volunteers participated in the study.
**Figures 4a** **and** **4b**: Effect of slowly L-cysteine releasing capsule (200 mg) in PPI treated ALDH2-deficient individuals on gastric juice acetaldehyde levels (a) and areas under the curve (b) after intragastric infusion of ethanol (15 vol%, 0.5 g/kg) (means f SEM).

### Description of embodiments

Based on prior art, alcohol-induced flushing and hypersensitivity reactions have been assumed to be caused by elevated levels of blood acetaldehyde resulting in enhanced release of histamine from the mast cells. However, after alcohol drinking, significantly higher acetaldehyde levels have been documented in saliva and gastric juice than in blood. Based on well documented experimentation and other pertinent data, the inventors of the present invention have reached the conclusion that acetaldehyde-mediated release of histamine from the tissue mast cells found in abundance in the upper digestive tract mucosa is the most plausible mechanism behind alcohol-induced flushing and hypersensitivity reactions.

According to the disclosure, the present invention thus relates to a slowly L-cysteine-releasing acetaldehyde-binding preparation for use in treating and preventing of alcohol flushing and alcohol-induced hypersensitivity reactions, as defined in the claims.

"Alcoholic drinks" are ethanol-containing drinks, the ethanol content varying within 2.8% by volume and 84% by volume.

"Alcoholic foodstuffs" and "non-alcoholic beverages" refer to foodstuffs containing less than 2.8% of ethanol. Such foodstuffs and beverages can be, for example, fermented juices or preserves, or foodstuffs preserved with small amounts of alcohol, pastries, jellies, and mousse seasoned with liqueur or corresponding preparations containing alcohol.

The use of the preparations according to the invention can be of benefit even, when light alcoholic drinks or foodstuffs are consumed, which contain small amounts of alcohol. Some foodstuffs can also already contain acetaldehyde. Acetaldehyde-containing foodstuffs, which have ethanol that is generated in connection with fermentation, such as beer, cider, wine, home-brewed beer, and other alcoholic drinks, as well as many juices. Of the alcoholic drinks, grappa and sherry contain particularly high concentrations of acetaldehyde.

Thus, according to a preferred embodiment of the present invention, the preparation is administered to the subject in connection with eating, i.e., just before, during or just after eating, or in connection with consuming alcohol, i.e., just before, during or after consuming a dose of alcohol.

"In connection with consuming alcohol" herein refers to the period of time that begins from the onset of alcohol intake and ends, when no more alcohol is present in the blood.

### Pharmaceutical preparation acting in the stomach

"A long-acting preparation that has a local effect on the stomach" refers to all monolithic or multi-particular tablets or capsules or granules as such, which, when wetted under the influence of the gastric juices adhere to the mucous membrane of the stomach or form a gel that floats in the contents of the stomach, as a consequence of which their residence time in the stomach is prolonged and thus enables a prolonged release in and a local effect of the drug on the stomach.

A special property required of the pharmaceutical composition that has a local effect on the stomach is that it remains in the stomach for as long as possible. Technically, this can be solved in two ways: by making a preparation that adheres to the mucous membrane of the stomach or making a preparation that floats in the contents of the stomach. The preparation can be rendered fixable to the mucous membrane of the stomach by using as additives cationic polymers, such as various chitosan grades. Preparations that float in the stomach are provided by using polymers (e.g., alginic acid) that form a gel and by adding to the preparation sodium hydrogen carbonate, which under the influence of gastric acid releases carbon dioxide, which in turn forms gas bubbles inside the gel. A liquid gel that floats in the stomach can also be prepared from sodium alginate, aluminium hydroxide, sodium hydrogen carbonate, and water, to which the acetaldehyde-binding compound, i.e. L-cysteine, can be added. A corresponding liquid preparation is also obtained by adding the acetaldehyde-binding substance to an aqueous dispersion of chitosan.

The amount of compound released in the conditions of the stomach is preferably 40-80 mg in an hour.

The preparation according to the invention, which releases in the stomach, has at least one - often two - polymers, which have the task of retaining the drug as long as possible, for two hours minimum, in the stomach either by attaching the preparation to the mucous membrane of the stomach or by forming a gel that floats in the contents of the stomach. Another function of the polymers is to prolong the release of the effective substance.

The preparation that locally binds acetaldehyde in the stomach can be a capsule comprising a mixture of powder or granules that forms a gel. In addition to the acetaldehyde-binding substances, the preparation comprises polymers that form a gel in the stomach, such as chitosans, alginates, sodium carboxymethylcellulose grades, carbomers or aluminium hydroxide. To advance floating in the stomach, the preparation can also comprise sodium hydrogen carbonate.

The amount of polymers in the preparation is 10-50%, preferably 15-40%, and most preferably 20-30%.

The proportion of sodium hydrogen carbonate can be 10-30%, preferably 20% of the amount of polymers.

The preparation that locally binds acetaldehyde in the stomach can be a granule preparation, wherein the acetaldehyde-binding substance is mixed with the fillers needed and, after that, granulated by using enteric polymers as binders. The binder used can be any known enteric polymer, preferably a polymer with a solution pH of 6-7, and most preferably the polymer is any of the methacrylate derivatives, which are known by the trade names Eudragit L and Eudragit S. The amount of enteric polymer in the preparation is preferably 2-5%, most preferably 3-4%.

The preparation that locally binds acetaldehyde in the stomach can be a liquid preparation, i.e., a mixture comprising, in addition to the acetaldehyde-binding substance, also sodium alginate, aluminium hydroxide, sodium hydrogen carbonate, and water. The amount of water in the whole preparation is 70-90%, most preferably about 75-85%. The amount of sodium alginate in the preparation is preferably 2-10%, most preferably about 5%, and the amount of aluminium hydroxide is preferably 5-15%, most preferably about 10%.

The relative composition of the preparation comprising granules can be as follows, for example:

| | | |
|---|---|---|
| Acetaldehyde-binding substances | 60 | parts |
| Chitosan | 10 - 40 | parts |
| Calcium hydrogen phosphate | 0 - 30 | parts |

The relative composition of the liquid preparation can be as follows, for example:

| | | |
|---|---|---|
| Acetaldehyde-binding substances | 10 | parts |
| Sodium alginate | 2 - 10 | parts |
| Aluminium hydroxide | 5 - 15 | parts |
| Sodium hydrogen carbonate | 1 - 2 | parts |
| Water | 70 - 80 | parts |

### Preparations for binding aldehydes in the mouth

One embodiment of the invention is a preparation that is to be placed in the mouth of a subject and contains L-cysteine that is intended for binding the aldehydes, such as the acetaldehyde, formaldehyde, acrylic aldehyde, propionaldehyde and butyraldehyde, in the mouth, and xylitol intended for destroying (i.e. killing) at least some of the microbes present in the mouth, as well as one or more non-toxic additives, which are harmless for human (or animal) consumption. Furthermore, xylitol has been shown to inhibit microbial acetaldehyde formation from ethanol (Uittamo et al. 2011).

The composition functions by the L-cysteine binding at least some of the aldehydes into a harmless form, whereas the xylitol kills or inhibits at least some of the microbes in the mouth that are responsible for generating aldehydes. Thus, the effect is synergistic, as a smaller amount of aldehydes will be produced, and an effective binding of said smaller amount of aldehydes is achieved, thus resulting in a considerably more efficient reduction of the aldehyde contents in the saliva compared to the contents achieved using prior solutions.

The term "additive" here includes carriers, fillers and binders, as well as aromatic agents colorants and non-functional additives. These additives are non-toxic, and preferably control the release of the active agents to take place specifically in the mouth, and most suitably in a sustained manner. These formulations are intended to be placed in the mouth, for example between the cheek or the lip and the gum, or they are intended to be sucked.

The composition comprises an effective amount of L-cysteine Here an effective amount means an amount capable of binding or inactivating the amount of aldehyde carried to the mouth from foodstuff, drinks or tobacco, or formed in the mouth, for example by the microbial activity therein, during the digestion of foodstuff or drinks in connection with or after the consumption.

The content of the cysteine is then preferably 1-50%, more preferably 5-40%, most suitably 20-30%, of the weight of the composition. Typically, the content is 20-25w-%.

The composition may also comprise an effective amount of the above mentioned xylitol. Here an effective amount means an amount capable of at least causing a measurable inactivation of the acetaldehyde-producing bacteria in the mouth or significantly inhibiting microbial acetaldehyde formation from alcohol.

Typically, a single unit, or formulation, of the composition comprises 50-500mg, preferably 50-300mg, more preferably 100-300mg, and most suitably 200-300mg of xylitol. However, as stated above, 1-2 units can be administered at once.

The content of xylitol is then preferably 10-90%, more preferably 10-60%, particularly 20-60%, and most suitably 40-60% of the weight of the composition. Typically, the content is about 50%.

### Pharmaceutical preparation acting in the large intestine

"A long-acting preparation that has a local effect in the large intestine" refers to all monolithic or multi-particular capsules or granules as such, which will not release the dose in a prolonged way until the preparation has drifted to the end of the small intestines or all the way to the large intestine.

The preparation according to the invention that releases acetaldehyde-binding substances in the large intestine in a prolonged way, carries the acetaldehyde-binding substance to the last part of the small intestine or to the large intestine before the substance in question is allowed to be released - whichever the releasing mechanism.

The pharmaceutical composition that binds acetaldehyde in the large intestine is administered orally. There are different techniques available for directing the release of an orally dosed drug to the large intestine. The most functional solutions are based on the use of enteric polymers. A film coating, which does not dissolve in the acidic environment of the stomach, but dissolves at pH 7 at the latest, can be made on the granules. In making the preparation, it is also possible to use polysaccharides that degrade under the effect of microbes of the large intestine, or polymers generated by azo bonds.

Useful enteric polymers include, for example, the grades of hydroxypropyl methylcellulose-acetatesuccinate (HPMC-AS) sold by the trade name Aqoat^{™}, Aqoat AS-HF^{™} in particular, a cellulose acetatephtalate (CAP) grade sold by the trade name Aquateric^{™}, and methacrylic acid-methylmethacrylate copolymers, the grade sold by the trade name Eudragit-S^{™} in particular.

The preparation according to the invention may have at least one ingredient, which adjusts the release of the effective substance not to take place until at the end of the small intestine or in the large intestine. This component can be a polymer that dissolves depending on the pH (=enteric polymer) or a polymer that degrades under the effect of the enzymes secreted by the bacteria of the large intestine. The polymer that controls the place of release can form a film around the entire preparation. It can also form a film around the particles (granules) contained by the multiple-part preparation. The polymer that degrades under the effect of the enzymes secreted by the bacteria of the large intestine can also be as a filler in a monolithic preparation, or as a filler in the granules or in a multiple-unit preparation prepared from these granules.

The preparation according to the invention can also be granules that comprise L-cysteine and are coated with an enteric film, the dissolution pH of the film-forming polymer being 6.0-7.5, preferably 6.5-7.0. The amount of film-forming enteric polymer of the entire mass of the granule is 5-30%, preferably 15-25%. The granule can comprise 20-40%, preferably about 30% of filler poorly soluble in water, such as calcium hydrogen phosphate.

The binder of the granule coated with the enteric film, according to the invention, can be an enteric polymer, the dissolution pH of which is 6.0-7.5, preferably 6.5-7.0. The amount of binder in the granule is 2-5%, preferably 3-4%.

The dosage unit of the pharmaceutical composition comprises 50-200 mg L-cysteine.

The amount of compound releasing in the conditions of the large intestine is preferably 50-100 mg in an hour.

### Slowly L-cysteine releasing preparations in the elimination of acetaldehyde

As evident from the present disclosure, cysteine, a semi-essential amino acid, is able to eliminate the toxicity of acetaldehyde by binding to it covalently. The inactive product is stable 2-methylthiazolidine-4-carboxylic acid (MTCA).

On the basis of the present invention, slowly L-cysteine releasing preparations can be used to eliminate the histamine-releasing effect of locally formed acetaldehyde in the digestive tract. This concept is supported by the following findings.

Orally administered slowly L-cysteine releasing preparations have been successfully used to eliminate free and active acetaldehyde in saliva during alcohol consumption and smoking (Salaspuro *et al.* 2002, 2006, Kartal *et al.* 2007). Furthermore, slowly L-cysteine releasing capsules (Acetium^{®} capsules) have been shown to eliminate effectively acetaldehyde in gastric juice of patients with atrophic gastritis after alcohol administration (Linderborg *et al.* 2011, Hellström *et al.* 2014).

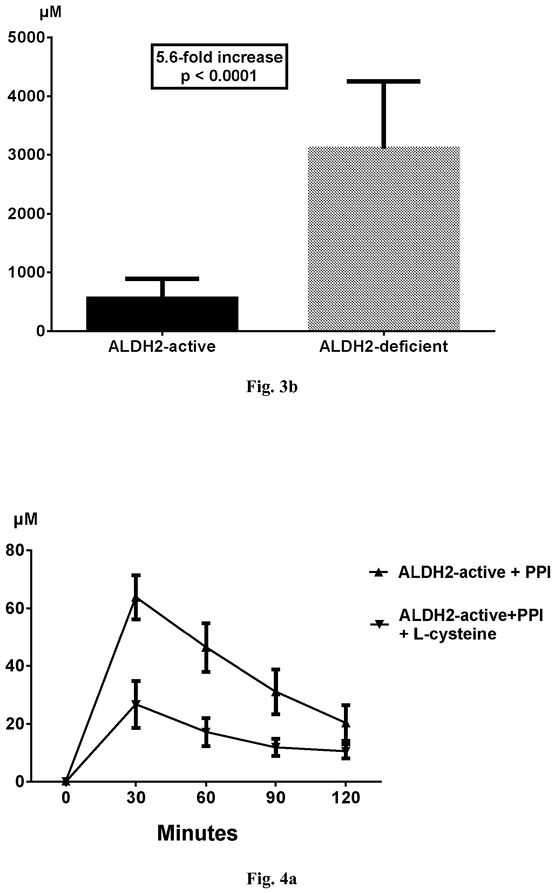

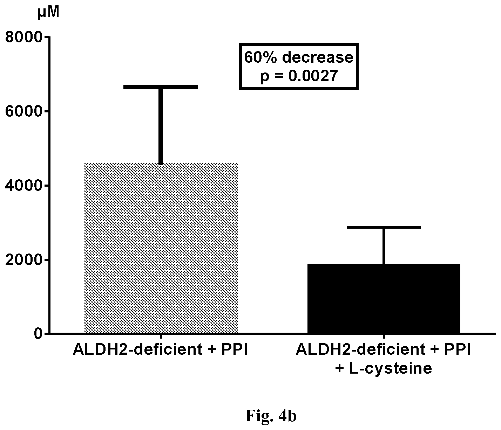

Most recently, it was found that after intragastric infusion of ethanol, gastric juice acetaldehyde levels in flushing ALDH2-deficient Japanese subjects are 5.6-fold higher as compared to those who have normal ALDH2-enzyme (Figures 3a and 3b). Most importantly, 60 % of gastric juice acetaldehyde could be eliminated by slowly L-cysteine releasing Acetium^{®} capsules (Figures 4a and 4b). The effect lasted for 2 hours.

Thereby, one embodiment of the present invention is a slowly L-cysteine releasing pharmaceutical preparation, for use in preventing and/or treating alcohol flushing and alcohol-induced hypersensitivity reactions. In this context, alcohol flushing is typically characterized by facial redness, increased skin temperature and heart rate and decreased diastolic blood pressure, and the alcohol induced hypersensitivity reactions are selected from allergic rhinitis (hay fever), allergic conjunctivitis, atopic dermatitis, eosinophilic esophagitis, anaphylaxis, chronic bronchitis and chronic obstructive pulmonary disease (COPD).

The pharmaceutical preparation is in the form of a capsule. A dosage regime of 1-2 slowly L-cysteine releasing capsules containing 50 - 200 mg L-cysteine taken 20 minutes before and/or immediately after intake of a dose of alcohol and repeating such dose at 1-2 hours intervals for as long as alcohol drinking continues is used. However, another equally good dosage regime is to use 1-2 slowly L-cysteine releasing capsules containing 50 - 200 mg L-cysteine taken 20 minutes before and/or immediately after intake of any food or beverage if there is any suspicion that the food or beverage contains any alcohol and/or acetaldehyde is used.

The cysteine releasing pharmaceutical preparation may also be in the form of a lozenge or a chewing gum or any other similar preparation, which is used together with cysteine-releasing capsules and following the previously described dosage regimes.

According to one embodiment the preparations for use according to the present invention include for example the commercial capsule preparations under trademark Acetium^{®} ( Biohit Oyj, Finland).

As disclosed earlier in the present application, the L-cysteine releasing preparation eliminates histamine-releasing effect of locally formed acetaldehyde in the digestive tract by swallowing, sucking or chewing slowly L-cysteine releasing preparations) and thus permitting L-cysteine to bind with acetaldehyde and inactivate it.

The method includes the steps of:
- swallowing 1-2 slowly L-cysteine releasing capsules containing 50 - 200 mg L-cysteine 20 minutes before drinking alcohol, and
- repeating such dose at 1-2-hour intervals for as long as alcohol drinking continues.

It should be understood by the skilled reader, however, that the embodiments given in the description above are for illustrative purposes only.

### References and related publications

IARC monographs on the evaluation of carcinogenic risks to humans. Personal habits and indoor combustions, volume 100E (2012).
Salaspuro MP. Acetaldehyde, microbes and cancer of the digestive tract. Crit Rev Clin Lab Sci 2003;40:183-208.
Salaspuro M. Acetaldehyde as a common denominator and cumulative carcinogen in digestive tract cancers. Scand J Gastroenterol 2009;44:912-25.
Salaspuro M. Acetaldehyde and gastric cancer. J Dig Dis 2011;12:51-9.
Kupari M, Eriksson CJ, Heikkilä J, Ylikahri R. Alcohol and the heart. Intense hemodynamic changes associating with alcohol flush in Orientals. Acta Med Scand 1983;213:91-98.
Väkeväinen S, Tillonen J, Salaspuro M. 4-methylpyrazole decreases salivary acetaldehyde levels in ALDH2-deficient subjects but not in subjects with normal ALDH2. Alcohol Clin Exp Res 2001;25:829-34.
Brooks PJ, Enoch M-A, Goldman D, Yokoyama A. The alcohol flushing response: An unrecognized risk factor for esophageal cancer from alcohol consumption. PLoS Medicine 2009;6:258-63.
Shimoda T, Kohno S, Takao A et al. Investigation of the mechanism of alcohol-induced bronchial asthma. J Allergy Clin Immunol 1996;97:74-84.
Koivisto T, Kaihovaara P, Salaspuro M. Acetaldehyde induces histamine release from purified rat peritoneal mast cells. Life Sci 1999;64:183-90.
Nihlen U, Greiff LJ, Nyberg P. et al. Alcohol-induced upper airway symptoms: prevalence and comorbidity. Respir Med 2005;99:726-9.
Linneberg A, Berg ND, Gonzales-Quintela A et al. Prevalence of self-reported hypersensitivity symptoms following intake of alcoholic drinks. Clin Exp Allergy 2008;38:145-51.
Miller NS, Goodwin DW, Jones FC et al. Antihistamine blockade of alcohol-induced flushing in Orientals. J Stud Alcohol 1988;49;16-20.
Myou S, Fujimura M, Nishi K et al. Inhibitory effect of terfenadine, a selective H1 histamine antagonist, on alcoholic beverage-induced bronchoconstriction in asthmatic patients. Eur Respir J 1995;8:619-23.
Takao A, Shimoda T, Matsuse H et al. Inhibitory effects of azelastine hydrochloride in alcohol-induced asthma. Ann Allergy Asthma Immunol 1999;82:390-4.
Linneberg A, Gonzales-Quintela A, Vidal C et al. Genetic determinants of both ethanol and acetaldehyde metabolism influence alcohol hypersensitivity and drinking behavior among Scandinavians. Clin Exp Allergy 2010;40:123-30.
Lindros KO, Stowell A, Pikkarainen P, Salaspuro M. Elevated blood acetaldehyde in alcoholics with accelerated ethanol elimination. Pharmacol Biochem Behav 1980;13 Suppl 1:119-24.
Homann N, Jousimies-Somer H, Jokelainen K, Heine R, Salaspuro M. High acetaldehyde levels in saliva after ethanol consumption: Methodological aspects and pathogenetic implications. Carcinogenesis 1997;18:1739-43.
Väkeväinen S, Tillonen J, Agarwal DP, Srivastava N, Salaspuro M. High salivary acetaldehyde after a moderate dose of alcohol in ALDH2-deficient subjects: Strong evidence for the local carcinogenic action of acetaldehyde. Alcohol Clin Exp Med 2000;24:873-77.
Dong YJ, Peng TK, Yin SJ. Expression and activities of class IV alcohol dehydrogenase and class III aldehyde dehydrogenase in human mouth. Alcohol 1996;13:257-62.
Mizoi Y, Iijiri I, Tatsuno Y et al. Relationship between facial flushing and blood acetaldehyde levels after alcohol intake. Pharmacol Biochem Behav 1979;10:303-11.
Chen Y-C, Lu R-B, Peng G-S et al. Alcohol metabolism and cardiovascular response in alcoholic patient homozygous for the ALDH2*2 variant gene allele. Alcohol Clin Exp Res 1999;23:1853-60.
Yokoyama A, Tsutsumi E, Imazeki H et al. Salivary acetaldehyde concentration according to alcoholic beverage consumed and aldehyde dehydrogenase-2 genotype. Alcohol Clin Exp Res 2008;32:1607-14.
Steer HW. Mast cells of the human stomach. J Anat 1976;121:385-97.
Syrjänen KJ. Morphologic manifestations of tumor-host relationships in association with breast, gastric and colorectal carcinoma. M.D. (Ph.D.) Thesis, University of Helsinki, pp. 1-86,1975. (ISBN-951-99064-3-6).
Gomes AP, Johann JE, Lovato GG, Ferreira AM. Comparative analysis of the mast cell density in normal oral mucosa, actinic cheilitis and lip squamous cell carcinoma. Braz Dent J 2008;19:186-9.
Sprince H, Parker CM, Smith GG, Gonzales LJ. Protective action of ascorbic acid and sulfur compunds against acetaldehyde toxicity: implications in alcoholism and smoking. Agents Actions 1976;5164-73.
Salaspuro V, Hietala J, Kaihovaara P, Pihlajarinne L, Marvola M, Salaspuro M. Removal of acetaldehyde from saliva by slow-release buccal tablet of L-cysteine. Int J Cancer 2002;97:361-4.
Salaspuro VJ, Hietala JM, Marvola ML, Salaspuro MP. Eliminating carcinogenic acetaldehyde by cysteine from saliva during smoking. Cancer Epid Biomark Prev 2006;15:146-49.
Kartal A, Hietala J, Laakso I, Kaihovaara P, Salaspuro V, Säkkinen M, Salaspuro M, Marvola M. Formulating and in-vivo evaluation of L-cysteine chewing gums for binding carcinogenic acetaldehyde in saliva during smoking. J Pharm Pharmacol 2007;59:1353-8.
Linderborg K, Marvola T, Marvola M, Salaspuro M, Färkkilä M, Väkeväinen S. Reducing carcinogenic acetaldehyde exposure in the achlorhydric stomach with cysteine. Alcohol Clin Exp Res 2011;35:516-22.
Hellström PM, Hendolin PH, Kaihovaara P et al. L-cysteine slow-release capsule formulation in prevention of gastric carcinogenesis associated with atrophic gastritis. Gastroenterology 2014;146 Suppl. 1, S-315, Sa1863.
Uittamo J, Nieminen MT, Kaihovaara P, Bowyer P, Salaspuro M, Rautemaa R. Xylitol onhibits carcinogenic acetaldehyde production by Candida species. Int J Cancer 2011;129:2038-41.

### Patent literature

WO 2012/027603
WO 2014/011676
US 2014/256760

## Claims

1. A slowly L-cysteine-releasing acetaldehyde-binding pharmaceutical preparation for use in preventing and treating alcohol flushing and alcohol-induced hypersensitivity reactions, selected from allergic rhinitis (hay fever), allergic conjunctivitis, atopic dermatitis, eosinophilic esophagitis, anaphylaxis, chronic bronchitis and chronic obstructive pulmonary disease (COPD), by eliminating histamine-releasing effect of locally formed acetaldehyde in the digestive tract, **characterized in that** a dosage regime of 1-2 slowly L-cysteine releasing capsules containing 50 - 200 mg L-cysteine taken 20 minutes before or immediately after intake of a dose of alcohol and repeating such dose at 1-2 hours intervals for as long as alcohol drinking continues is used.

2. The preparation for use according to claim 1 in preventing and treating alcohol flushing and alcohol-induced hypersensitivity reactions, wherein alcohol flushing is **characterized by** facial redness, increased skin temperature and heart rate and decreased diastolic blood pressure.

3. The preparation for use according to claim 1 or 2 in preventing and treating alcohol flushing and alcohol-induced hypersensitivity reactions, **characterized in that** a dosage regime of 1-2 slowly L-cysteine releasing capsules containing 50 - 200 mg L-cysteine taken 20 minutes before or immediately after intake of any food or beverage if there is any suspicion that the food or beverage contains any alcohol and/or acetaldehyde is used.

## Patentansprüche

1. Langsam L-Cystein freisetzende, acetaldehydbindende pharmazeutische Zubereitung zur Verwendung beim Verhindern und Behandeln von Alkohol-Flushing und alkoholinduzierten Überempfindlichkeitsreaktionen, ausgewählt aus allergischer Rhinitis (Heufieber), allergischer Konjunktivitis, atopischer Dermatitis, eosinophiler Ösophagitis, Anaphylaxie, chronischer Bronchitis und chronisch obstruktiver Lungenerkrankung (COPD) durch Eliminieren von histaminfreisetzendem Effekt von lokal gebildetem Acetaldehyd im Verdauungstrakt, **dadurch gekennzeichnet, dass** ein Dosierungsregime von 1-2 langsam L-Cystein freisetzenden Kapseln, die 50 - 200 mg L-Cystein enthalten, 20 Minuten vor oder unmittelbar nach Einnahme einer Alkoholdosis genommen, und Wiederholen solcher Dosis in Intervallen von 1-2 Stunden, solange Trinken von Alkohol fortgesetzt, angewendet wird.

2. Zubereitung zur Verwendung nach Anspruch 1 beim Verhindern und Behandeln von Alkohol-Flushing und alkoholinduzierten Überempfindlichkeitsreaktionen, wobei Alkohol-Flushing **gekennzeichnet ist durch** Gesichtsröte, erhöhte Hauttemperatur und Herzfrequenz und verringerten diastolischen Blutdruck.

3. Zubereitung zur Verwendung nach Anspruch 1 oder 2 beim Verhindern und Behandeln von Alkohol-Flushing und alkoholinduzierten Überempfindlichkeitsreaktionen, **dadurch gekennzeichnet, dass** ein Dosierungsregime von 1-2 langsam L-Cystein freisetzenden Kapseln, die 50 - 200 mg L-Cystein enthalten, 20 Minuten vor oder unmittelbar nach Einnahme von beliebigem Lebensmittel oder Getränk, wenn es eine Vermutung gibt, dass das Lebensmittel oder Getränk Alkohol und/oder Acetaldehyd enthält, angewendet wird.

## Revendications

1. Préparation pharmaceutique liant l'acétaldéhyde à la L-cystéine à libération lente destinée à être utilisée dans la prévention et le traitement de réactions d'intolérance à l'alcool et d'hypersensibilité induite par l'alcool, choisies parmi la rhinite allergique (rhume des foins), la conjonctivite allergique, la dermatite atopique, l'oesophagite à éosinophiles, l'anaphylaxie, la bronchite chronique et la bronchopneumopathie chronique obstructive (BPCO), en éliminant l'effet de libération d'histamine de l'acétaldéhyde formé localement dans l'appareil digestif, **caractérisée en ce qu'**un schéma posologique de 1 à 2 gélules de L-cystéine à libération lente contenant de 50 à 200 mg de L-cystéine prises 20 minutes avant ou immédiatement après l'ingestion d'une dose d'alcool et la répétition de cette dose à 1 à 2 heures d'intervalle est utilisé pendant toute la durée de la consommation d'alcool.

2. Préparation destinée à être utilisée selon la revendication 1 dans la prévention et le traitement de réactions d'intolérance à l'alcool et d'hypersensibilité induite par l'alcool, dans laquelle l'intolérance à l'alcool est **caractérisée par** des rougeurs du visage, une augmentation de la température de la peau et de la fréquence cardiaque et une diminution de la pression artérielle diastolique.

3. Préparation destinée à être utilisée selon la revendication 1 ou 2 dans la prévention et le traitement de réactions d'intolérance à l'alcool et d'hypersensibilité induite par l'alcool, **caractérisée en ce qu'**un schéma posologique de 1 à 2 gélules de L-cystéine à libération lente contenant de 50 à 200 mg de L-cystéine prises 20 minutes avant ou immédiatement après l'ingestion de nourriture ou d'une boisson est utilisé s'il existe le moindre doute concernant l'aliment ou la boisson susceptible de contenir de l'alcool et/ou de l'acétaldéhyde.
